(19) Europäisches Patentamt
European Patent Office
Office européen des brevets

(11) **EP 0 834 738 B1**

(12) **EUROPEAN PATENT SPECIFICATION**

(45) Date of publication and mention
of the grant of the patent:
**26.07.2006 Bulletin 2006/30**

(51) Int Cl.:
***G01N 27/12*** (2006.01)

(21) Application number: **97917410.9**

(22) Date of filing: **17.04.1997**

(86) International application number:
**PCT/JP1997/001321**

(87) International publication number:
**WO 1997/039342 (23.10.1997 Gazette 1997/45)**

(54) **GAS SENSOR AND METHOD OF MANUFACTURING THE SAME**

GASSENSOR UND DESSEN HERSTELLUNGSVERFAHREN

DETECTEUR DE GAZ ET PROCEDE DE FABRICATION

(84) Designated Contracting States:
**DE FR GB IT NL**

(30) Priority: **18.04.1996 JP 9703696**
**18.04.1996 JP 9704596**

(43) Date of publication of application:
**08.04.1998 Bulletin 1998/15**

(73) Proprietor: **MIKUNI CORPORATION**
**Chiyoda-ku,**
**Tokyo 101-0021 (JP)**

(72) Inventors:
• **HASUMI, Kazuhisa**
**Kanagawa 250 (JP)**
• **NAGANO, Kentaro**
**Kanagawa 241 (JP)**
• **HORIUCHI, Hideyuki**
**Kanagawa 250 (JP)**
• **OKADA, Osamu**
**Osaka 589 (JP)**

(74) Representative: **Pearson, James Ginn et al**
**Abel & Imray,**
**20 Red Lion Street**
**London WC1R 4PQ (GB)**

(56) References cited:
**EP-A- 0 440 858     JP-A- 6 258 270**
**JP-A- 7 140 100     JP-A- 52 060 695**
**JP-A- 56 033 535**

EP 0 834 738 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Technical Field

The present invention is utilized for the detection of carbon monoxide.

### Background Art

Sensors that make use of ceramic semiconductor materials to detect gases present in air have been known for some time. Whereas hitherto only sensors utilizing reactions involving n-type ceramic semiconductors were known, the inventors associated with the present application have discovered that gases can be detected using high-purity CuO, which is a p-type semiconductor. They have previously filed a patent application for this, which application will hereinafter be referred to as "the prior application." This prior application was laid open to public inspection as Japanese Kokai Patent 6-258270. In the gas sensor disclosed in this prior application, at least 99 wt% of the semiconductor component contributing to conductivity in the sintered product was CuO, and the amount of additive was no more than 1 wt%. It was also disclosed in the prior application that CO sensitivity is increased by adding an alkaline metal compound as this additive which is present at no more than 1 wt%.

However, although CO sensitivity was increased by adding an alkali metal compound to the high-purity CuO, the CO sensitivity thus obtained was only about twice the sensitivity to the same concentration of $H_2$ for example. A further consideration is that recent research on the connection between global warming and $CO_2$ gas has highlighted the need for a gas sensor capable of measuring $CO_2$ gas concentrations.

Meanwhile, detection of gases in exhaust gases differs from detection in air in that the partial pressure of oxygen varies, and $CO_2$ concentration and water vapor partial pressure vary according to the state of combustion. Under such conditions there are no sensors with high enough sensitivity to CO alone that they can be used for CO detection without some modification. For example, a gas sensor comprising CuO with addition of $Na_2CO_3$ is sensitive to $CO_2$ as well, and at the $CO_2$ concentrations present in exhaust gas from gas-fired water heaters, its sensitivity to $CO_2$ is close to its sensitivity to CO at the CO concentrations which have to be detected to give warning of a dangerous amount of CO. This means that selective detection of CO will sometimes be unsuccessful.

For example, in experiments performed by the present inventors, it was found that at a $CO_2$ concentration of 5.5% an experimental gas sensor comprising CuO with addition of $Na_2CO_3$ gave about the same detection output for $CO_2$ as the output in response to the approximately 2000-4000 ppm of CO which was generated during incomplete combustion. Given than both $CO_2$ and CO are present in exhaust gas during incomplete combustion, and that even during normal combustion $CO_2$ is generated at concentrations sufficiently large to be expressed in percent rather than ppm, it will be seen that such high sensitivity to $CO_2$ is unsuitable for selective detection of CO.

It is an object of the present invention to solve such problems by providing gas sensors capable of selective detection of CO and $CO_2$; gas sensors in which the $CO_2$ sensitivity has been made lower than the CO sensitivity; and manufacturing methods for these sensors.

### Disclosure of Invention

The increase in CO gas selectivity due to adding a sodium compound to CuO, which is a p-type semiconductor, is disclosed in the prior application. Nevertheless, when the prior application was filed it was thought that gas detection by means of a structure in which electrodes are attached to a p-type semiconductor consisting mainly of CuO was possible only if high-purity CuO was used, i.e., CuO in which additive-derived semiconductor constituents other than CuO amount to no more than 1 wt%.

However, subsequent research has revealed that gas detection is still possible when the amount of additive is increased.

The present invention is a gas sensor in which the $CO_2$ sensitivity has been made lower than the CO sensitivity, said gas sensor having a p-type member formed from a p-type semiconductor the main constituent of which is CuO and to which a sodium compound has been added, and two electrodes which are connected to this p-type member, said electrodes serving to extract changes in electrical characteristics resulting from the presence of a gas to be detected, wherein the sodium salt of tungstic acid or molybdic acid is included as the sodium compound. The content of the sodium compound is from 0.5 to 23 wt% as tungsten relative to CuO when a sodium salt of tungstic acid is used, and 0.4 to 16 wt% as molybdenum relative to CuO when a sodium salt of molybdic acid is used.

According to research by the present inventors, if the sodium salt of tungstic acid or molybdic acid is added, $CO_2$ sensitivity decreases relative to CO sensitivity. By utilizing such materials in gas sensors, CO gas can be selectively detected in the exhaust gas discharged by gas-fired water heaters and other combustion equipment, and incomplete combustion can be detected.

The present invention also provides a method for manufacturing such gas sensors by forming a member with electrical characteristics which change in accordance with the presence of a gas to be detected, said member being formed by adding a sodium compound to CuO and then molding and firing, wherein a sodium salt of tungstic acid or a sodium salt of molybdic acid upon firing is added as the sodium compound. The content of the sodium compound is from 0.5 to 23 wt% as tungsten relative to CuO when a sodium salt of tungstic acid is used, and 0.4 to 16 wt% as molybdenum relative to CuO when a sodium salt of molybdic acid is used.

In experiments performed by the present inventors, when $Na_2WO_4 \cdot 2H_2O$ or $Na_2MoO_4 \cdot 2H_2O$ was used as the added substance, good results were obtained at additions of 1 to 40 wt% relative to CuO. These additions are equivalent to 0.5 to 23 wt% of tungsten relative to CuO, and 0.4 to 16 wt% of molybdenum relative to CuO. If the amount of addition exceeded 40 wt%, the sintered product did not maintain a solid shape and could not be used as a sensor. In particular, if the sintered product is used as a mass, then for both $Na_2WO_4 \cdot 2H_2O$ and $Na_2MoO_4 \cdot 2H_2O$ an addition of 20 wt% ensured that it could readily be handled without breaking.

In these cases as well it is preferable for the maximum temperature during firing to be at least 400°C and no more than 860°C. In experiments by the present inventors, the sintered product could be readily handled without breaking if the maximum temperature during firing was at least 500°C. In addition, sensitivity to co-present gases could be kept low by ensuring the maximum temperature during firing was at least 500°C and no more than 850°C.

The sintered mass can be used as a gas sensor either just as it is, or after machining to a suitable size. Alternatively, it can be formed as a thick film. A thick film can also be formed by grinding the sintered mass described above and using this as the raw material.

The powdered CuO used as the raw material preferably has primary particles with a specific surface area of at least 2 $m^2/g$, and more preferably of at least 20 $m^2/g$. A specific surface area of at least 2 $m^2/g$ is equivalent to a particle size of 1 $\mu$m or less, while a specific surface area of at least 20 $m^2/g$ is equivalent to a particular size of 0.25 $\mu$m or less. In experiments performed by the inventors, when CuO with a specific surface area of less than 2 $m^2/g$ was used, subsequent sintering did not go to completion and the sintered product obtained ended up being easily broken. It is therefore though that a particular gas sensor cannot be obtained using CuO with such a small specific surface area.

**Brief Description of Drawings**

FIG. 1 to FIG. 3 show a first embodiment of the present invention, with FIG. 1 being a plane view, FIG. 2 a side view and FIG. 3 a rear plane view.

FIG. 4 and FIG. 5 show a second embodiment of the present invention, with FIG. 4 being a plane view and FIG. 5 a sectional view.

FIG. 6 to FIG. 9 show several ways of utilizing a gas sensor in which a sodium salt of tungstic acid or molybdic acid has been added to CuO.

FIG. 10 shows the configuration of apparatus for measuring sensitivity to gases.

FIG. 11 shows, for reference, an example of measurement results.

FIG. 12 to FIG. 14 show a gas sensor used for measurements, with FIG. 12 being a perspective view, FIG. 13 a side view and FIG. 14 a view facing one of the electrodes.

FIG. 15 gives an example of the results of measurements of sensitivity ratio for various gases.

FIG.16 gives measurements showing the dependence of gas sensitivity on the amount of $Na_2CO_3$ addition.

FIG.17 shows the dependence of sensitivity ratio on the specific surface area of the CuO used as the raw material.

FIG.18 shows the relation between sensitivity and the specific surface area of the CuO raw material.

FIG.19 shows the relation between particle size and specific surface area of CuO.

FIG.20 shows the relation between firing temperature and sensitivity.

FIG.21 shows the relation between firing temperature and the density of the sintered product obtained.

FIG.22 shows an example of the results of measurements of the sensitivity to various test gases of a gas sensor fabricated by adding $Na_2WO_4 \bullet 2H_2O$ to CuO.

FIG.23 shows an example of the results of measurements of changes in the terminal voltage of a gas sensor in a gas composition simulating the exhaust gases of a water heater during incomplete combustion.

FIG.24 expresses the measurement results of FIG.23 as changes in sensitivity.

FIG.25 shows an example of sensor output characteristics during incomplete combustion in a gas-fired water heater of forced draft type.

FIG.26 shows sensor resistance as a function of CO concentration.

FIG.27 shows sensor resistance as a function of $H_2$ concentration.

FIG.28 shows sensor resistance as a function of water vapor concentration.

FIG.29 shows sensor resistance as a function of $H_2$ concentration.

FIG.30 shows sensor resistance as a function of NO concentration.

FIG.31 shows the results of measurements of sensitivity to CO, $H_2$, $CH_4$ and $C_3H_8$.

FIG.32 shows the results of measurements of sensitivity to various test gases.

FIG.33 shows the results of measurements of gas sensitivity as a function of maximum temperature during firing.

FIG.34 gives an example of the results of measurements of gas sensitivity as a function of firing temperature.

FIG. 35 gives an example of the results of measurement of gas sensor sensitivity as a function of the amount of $Na_2WO_4$ addition.

FIG. 36 gives an example of the results of measurements of resistance as a function of test gas, for a gas sensor in which $Na_2MoO_4 \cdot 2H_2O$ has been added to CuO.

FIG. 37 gives an example of the results of measurements when the structure of the gas sensor used for FIG. 28 was changed.

FIG. 38 gives an example of the results of measurements of sensitivity as a function of the amount of $Na_2MoO_4$ added.

**Description of a specific embodiment**

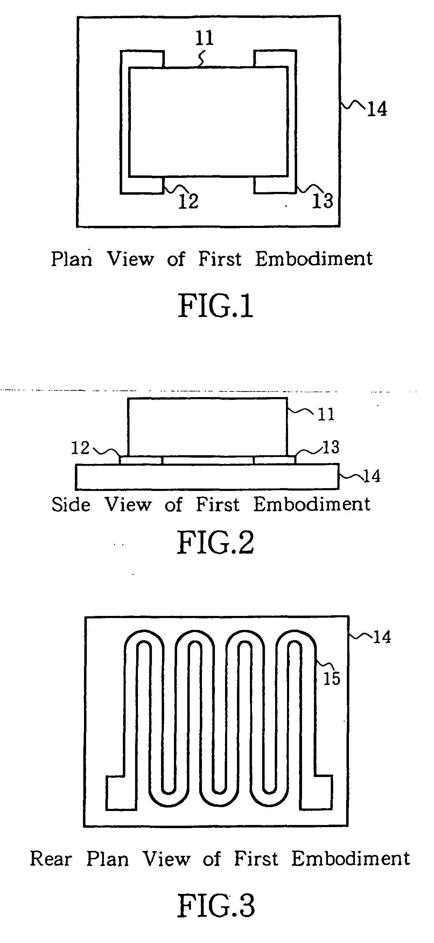

FIG. 1 to FIG. 3 show a first embodiment of a gas sensor according to the present invention. FIG. 1 is plane view, FIG. 2 a side view and FIG. 3 a rear plane view. This gas sensor has a construction in which Au electrodes 12 and 13 are formed on ceramic substrate 14, and p-type member 11 comprising a sintered mass is in a contact with these Au electrodes 12 and 13. P-type member 11 is formed from a p-type semiconductor whereof the main constituent is CuO, and the two Au electrodes 12 and 13 are connected to this p-type member 11 so that they can extract changes in electrical characteristics resulting from the presence of a gas to be detected. Heater 15 for heating can be provided on the rear of ceramic substrate 14 so that this gas sensor can be heated. When operating, a voltage is applied between Au electrodes 12 and 13, and changes in voltage or current, or in voltage and current, between Au electrodes 12 and 13 due to the presence of a gas to be detected are monitored.

A p-type semiconductor containing the sodium salt of at least one acid selected from the group comprising tungstic acid and molybdic acid is used as p-type member 11.

The p-type semiconductor containing a sodium salt of tungstic acid or molybdic acid as an additive is obtained by mixing with CuO as sodium compound of the sort that such an additive will be obtained by firing, and then molding and firing this. The CuO used as the raw material has primary particles with a specific surface area of at least 2 $m^2$/g and preferably of at least 20 $m^2$/g, and with a particle size of no more than 1μm and preferably of no more than 0.25 μm. The maximum temperature during firing is at least 400°C and preferably no more than 860°C, and more preferably still at least 500°C and no more than 700°C. Bulk density can be controlled and CO selectivity increased by appropriate selection of the particle shape of the CuO raw material powder, and of the maximum firing temperature.

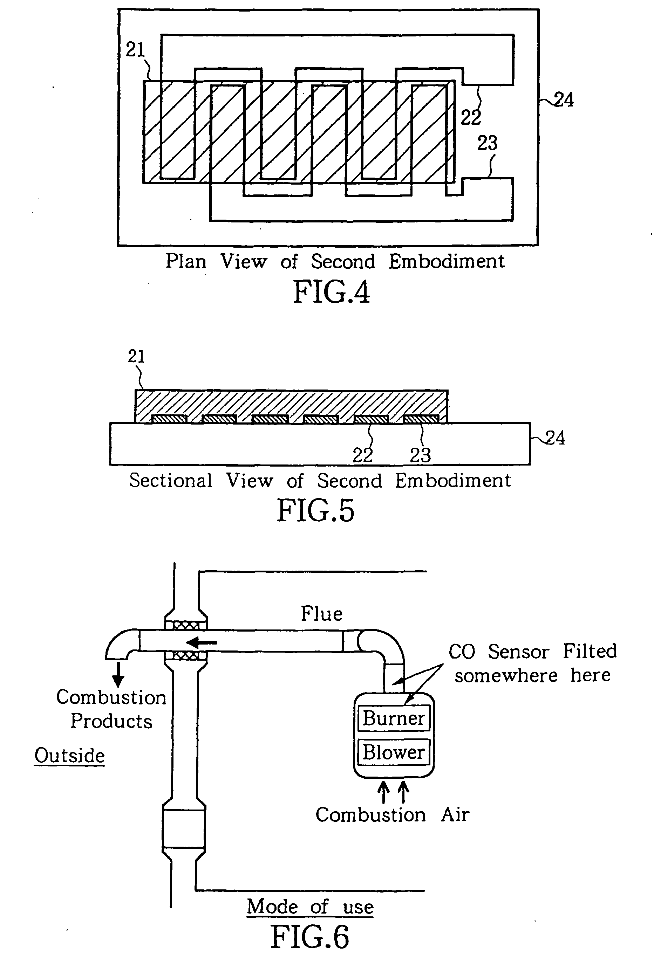

FIG. 4 and FIG. 5 show a second embodiment of a gas sensor according to the present invention. FIG. 4 is a plane view, and FIG. 5 is sectional view.

This gas sensor comprises interdigitated electrodes 22 and 23 which are formed on ceramic substrate 24, and p-type member 21 which as been formed as a thick film over these electrodes. P-type member 21 is formed by firstly printing a paste so as to be in contact with interdigitated electrodes 22 and 23, said paste having as its main solid constituents powdered CuO and an additive, the additive being a sodium compound which becomes a sodium salt of tungstic acid or molybdic acid as a result of firing. This paste is then fired. Although not illustrated, as in the first embodiment a heater can be provided on the rear surface of ceramic substrate 24 in order to heat this gas sensor. It is also possible to form a thick film in the same manner as in the first embodiment, by using as the raw material the product obtained by grinding the sintered mass. Alternatively, a thick film can be formed by mixing the sodium compound with powdered CuO, dispersing the mixture in an organic solvent, using screen printing or other method to print the paste obtained, and then firing this printed paste.

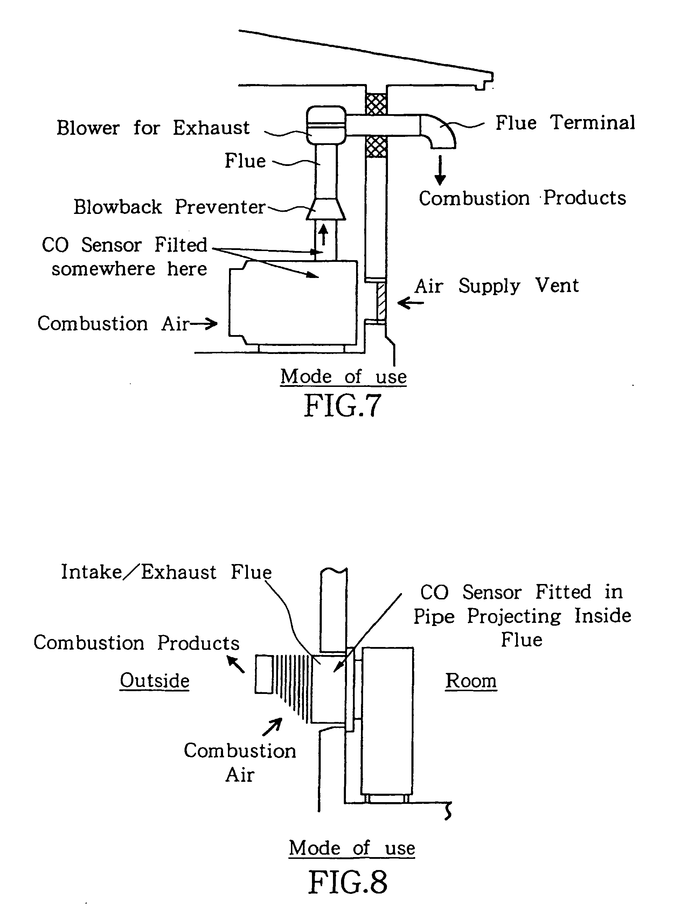

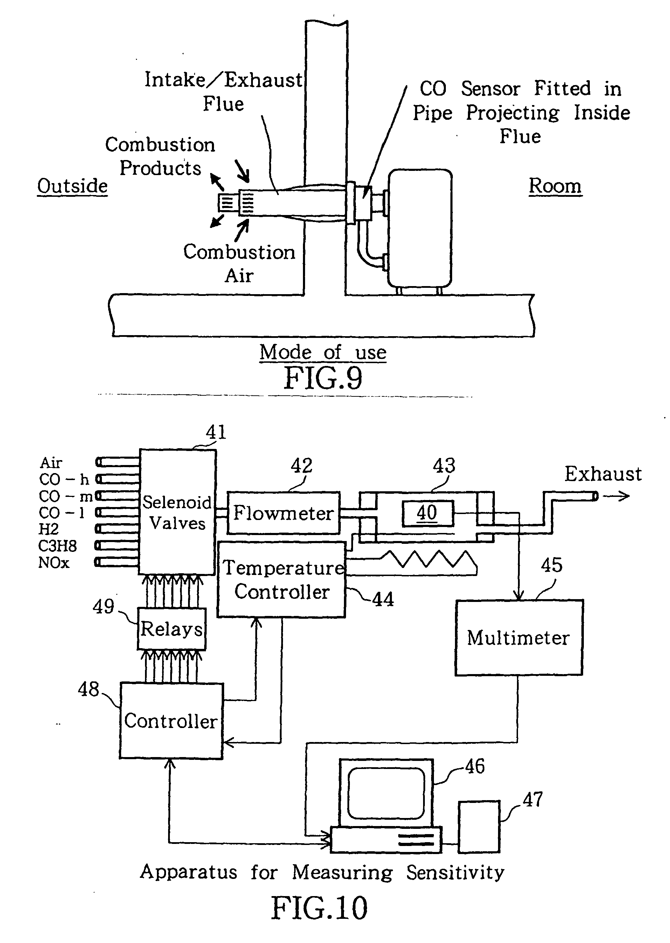

FIG. 6 to FIG. 9 show several ways for utilizing a gas sensor in which a sodium salt oftungstic acid or molybdic acid has been added to CuO. A gas sensor in which $Na_2CO_3$ has been added to CuO is capable of selective detection of CO and $CO_2$, and it is assumed that such sensors will mainly be used in the air. As opposed to this, because a gas sensor in which a sodium salt oftungstic acid or molybdic acid has been added to CuO has a $CO_2$ sensitivity which is relatively low compared with its CO sensitivity, and is capable of selective detection of CO gas in the exhaust gases discharged from combustion equipment, its utilization will involve being fitted inside, or to the exhaust system of, various types of combustion equipment. FIG.6 shows forced draft type combustion equipment which uses a blower to feed room air to the burner and to discharge exhaust gas to the outside. FIG. 7 shows forced exhaust type combustion equipment which uses air taken in from the room and from outside for combustion, and discharges exhaust gases to the outside by means of a blower. FIG. 8 shows natural feed and exhaust type combustion equipment which takes in outside air for combustion and discharges the exhaust gases to the outside. FIG.9 shows forced feed and exhaust type combustion equipment which takes in outside air and forcibly discharges exhaust gases.

The results of measurements of experimental gas sensors will now be explained.

FIG. 10 shows the configuration of apparatus for measuring sensitivity to gases. This measuring apparatus is operated as follows. Gas sensor 40 to be tested is placed inside quartz tube 43 and a flow of air plus a gas to be detected, such as CO or $H_2$, is passed into this quartz tube 43 via solenoid valves 41 and mass flowmeter 42. The temperature of the gas flow is controlled by temperature controller 44. The voltage applied to gas sensor 40 and the current flowing through it are measured by multimeter 45, and the measured values are processed by personal computer 46 and stored in external storage device 47. Solenoid valves 41 are configured to be capable of selecting the gas to be detected, adding it to air, and supplying the mixture to quartz tube 43. They are operated by control signals supplied from controller 48 via relays 49. When personal computer 46 has acquired the value of the current detected by multimeter 45, after a suitable time has elapsed it outputs to controller 48 a control signal for switching over the gas being supplied. The operating temperature of gas sensor 40 being measured is set to 230-260°C. If a heater has been provided on gas sensor 40, this operating temperature is controlled by this heater. If a heater is not provided, the operating temperature is controlled from outside quartz tube 43.

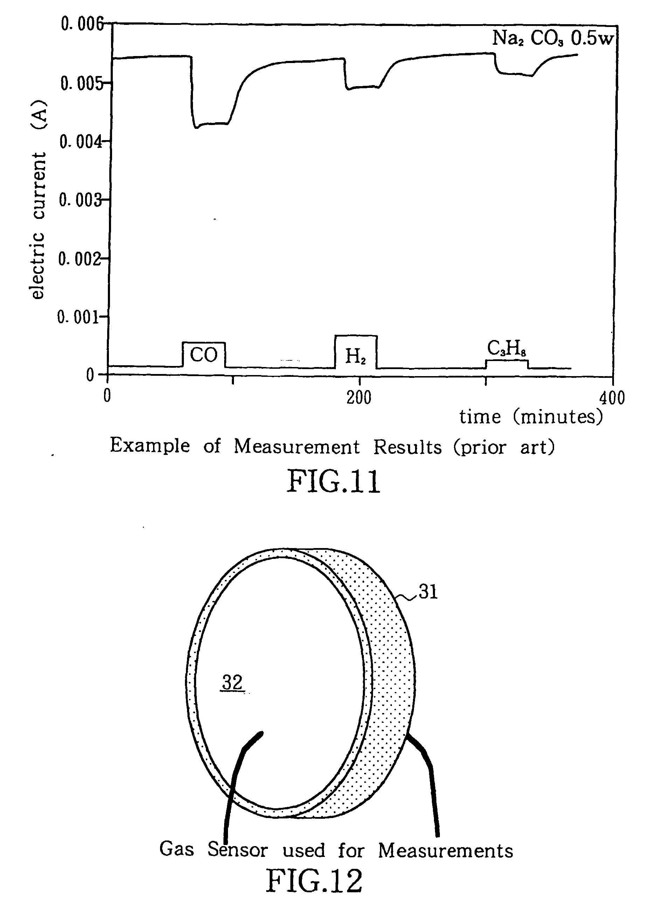

FIG. 11 shows an example of the results of measurements obtained by multimeter 45 as shown in FIG. 6 of the prior application. In this example, a sintered mass comprising 0.5 wt% addition of $Na_2CO_3$ to CuO was maintained at 260°C, a fixed voltage applied, and CO, $H_2$ and $C_3H_8$ supplied as test gases to be detected. In each case, the supplied gas flow consisted of 4000 ppm of the test gas in air. When each test gas was introduced, a change in electric current was observed. The supply of the test gas was then discontinued, and after the current has stabilized again, the next test gas for detection was introduced. The larger the current fluctuation, the higher the sensitivity to the gas being detected.

However, although the difference in sensitivity of a given gas sensor to each test gas can be indicated by comparing the sizes of the current fluctuations, comparisons cannot be made with other gas sensors. Accordingly, in the present specification the value defined as:

$$[(R_{gas}/R_0) - 1] \times 100$$

is used to indicate sensitivity, where $R_0$ is resistance in the air and $R_{gas}$ is resistance under a flow of the gas to be detected. Alternatively, the value defined as:

$$[(R_{gas}/R_{base}) - 1] \times 100$$

is used to indicate sensitivity when use in a location other than in the air is being considered. Here, $R_{base}$ is resistance in the base gas and $R_{gas}$ is resistance under a flow of the gas to be detected.

"Sensitivity ratio" will be used to express gas selectivity. For each test gas, this sensitivity ratio is the sensitivity to that gas normalized by the sensitivity to a particular test gas. For a given gas sensor, this sensitivity ratio coincides with the ratio of current change at a particular voltage.

**Measurement Example 1 (Comparative)**

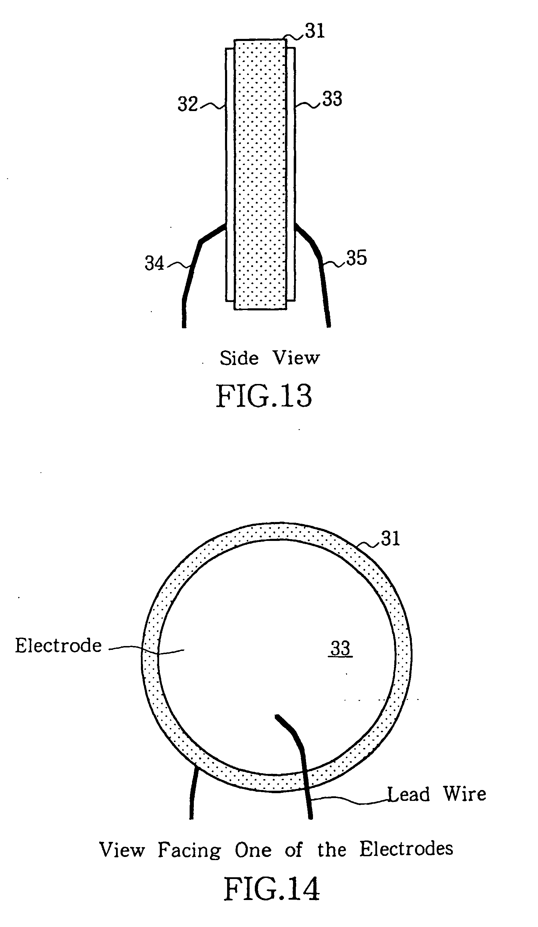

A gas sensor was fabricated by attaching electrodes to the sintered mass obtained by adding 10 wt% of $Na_2CO_3$ to CuO with primary particles having a specific surface area of 2.36 $m^2$/g, and then firing in air at 700°C for 30 minutes. FIG.12 to FIG. 14 show the structure of this gas sensor. FIG.12 is a perspective view, FIG. 13 a side view, and FIG. 14 a view facing one of the electrodes. This gas sensor has a structure wherein sintered mass 31 is sandwiched between electrodes 32 and 33. Lead wires 34 and 35 are attached to electrodes 32 and 33 respectively. Mass 31 was 2.3 mm thick and its lateral surface area was approximately 50 $mm^2$.

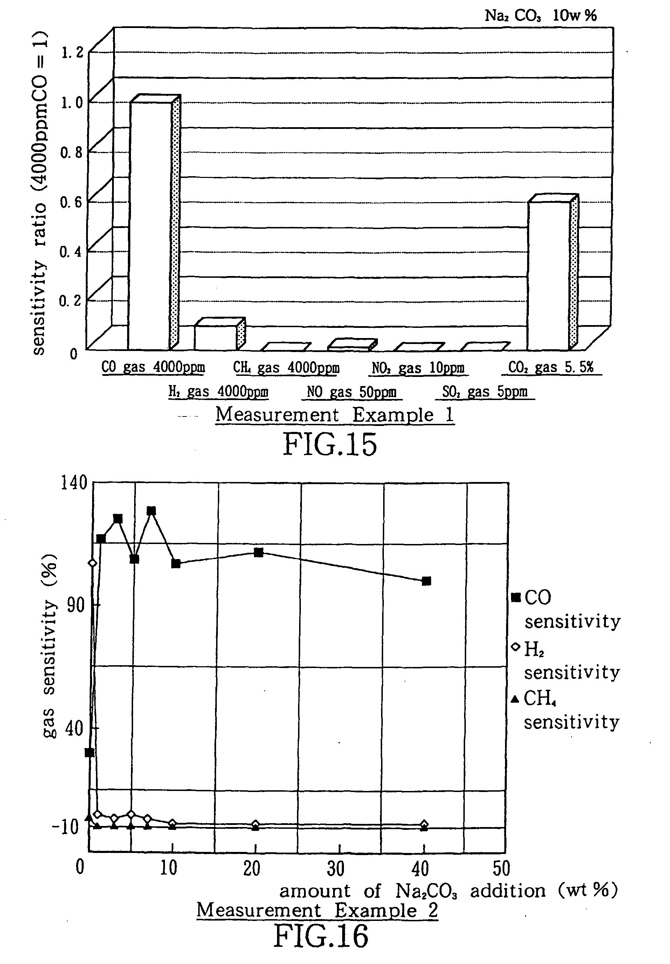

FIG.15 gives an example of the results of measurements of sensitivity ratio for various gases. For these measurements, 0.1 V was applied between electrodes 32 and 33. The sensitivity ratio for each test gas being detected was then obtained by taking the current change measured for 4000 ppm of CO as "1" and normalizing the current change measured for each test gas. In addition to CO, the following gases were tested: $H_2$, $CH_4$ (as a representative combustible gas which is present in air), NO and $NO_2$ as nitrogen oxides, $SO_2$ as a sulphur oxide, and $CO_2$. The following concentrations were used for the test gases: 4000 ppm for $H_2$ and $CH_4$; 50 ppm for NO; 10 ppm for $NO_2$; 5 ppm for $SO_2$; and 5.5% for $CO_2$. The base gas was air. It was found that the selectivity for CO was high, with the sensitivity ratio of $H_2$ relative to CO being approximately 1/10. It was also found that there was considerable sensitivity to $CO_2$ as well.

**Measurement Example 2 (Comparative)**

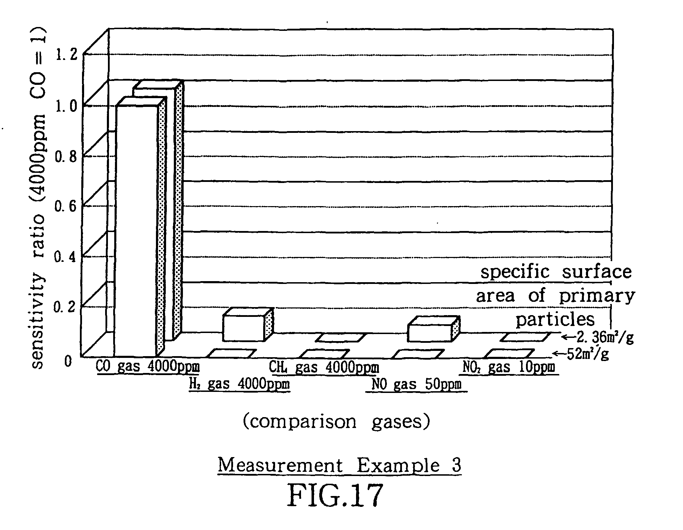

Gas sensors similar to that of Measurement Example 1 were fabricated with different amounts of $Na_2CO_3$ addition, and measurements made of variations in sensitivity to CO, $H_2$ and $CH_4$. The results of these measurements are given in FIG.16. When the amount of $Na_2CO_3$ addition exceeded 1 wt%, sensitivity to CO became high, but sensitivity to the other gases became very low.

**Measurement Example 3 (Comparative)**

Gas sensors similar to that of Measurement Example 1 were fabricated using as the raw material CuO of different specific surface areas, and measurements made of variations in sensitivity ratio. An example of the results of these measurements is given in FIG.17. This figure shows some of the measurement results already given in FIG.15, side by side with the results of similar measurements made on a sintered mass fired under the same conditions but using as the raw material CuO having primary particles with a specific surface area of 52 $m^2$/g. It will be seen that although the additive and firing conditions were identical, the sensitivity ratios of both $H_2$ and NO were lower as a result of using as the raw material a CuO with a larger specific surface area.

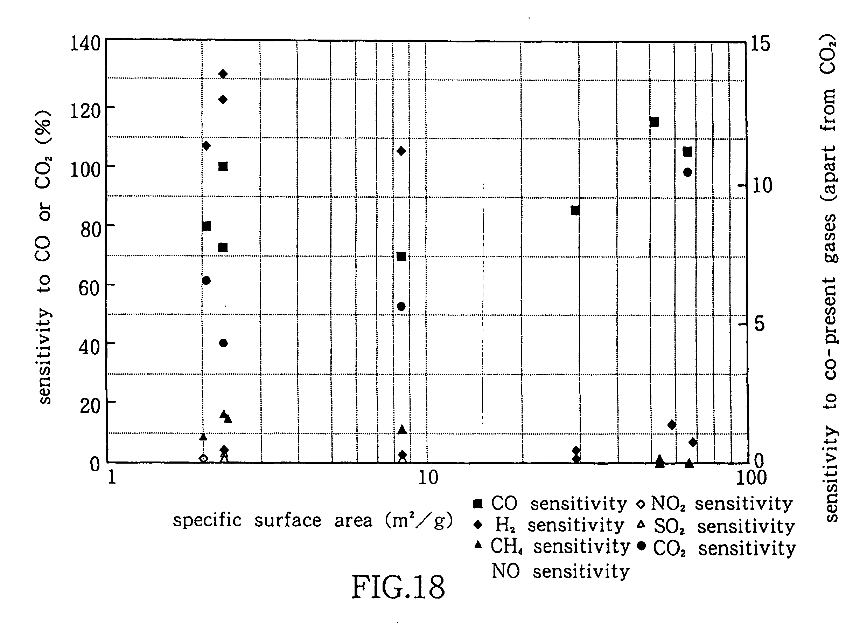

FIG.18 shows the relation between sensitivity and the specific surface area of the CuO raw material. CO, $H_2$, $CH_4$, NO, $NO_2$, $SO_2$ and $CO_2$ were used as the test gases, at concentrations of 4000 ppm, 4000 ppm, 4000 ppm, 50 ppm, 10 ppm, 5 ppm and 5.5%, respectively. The base gas was air. The vertical axis on the left-hand side of FIG.18 shows the sensitivity to CO and $CO_2$, while the vertical axis on the right-hand side shows the sensitivity to the other co-present gases. Note that the scale of these two axes differs by a factor of approximately 10. It was found that the larger the specific surface area of the primary particles of powdered CuO used as the raw material, the lower the sensitivity to gases other than CO and $CO_2$. In particular, when the specific surface area exceeded 10-20 $m^2$/g, whereas the sensitivity to CO and $CO_2$ increased, the sensitivity to other gases such as $H_2$ underwent a marked decrease.

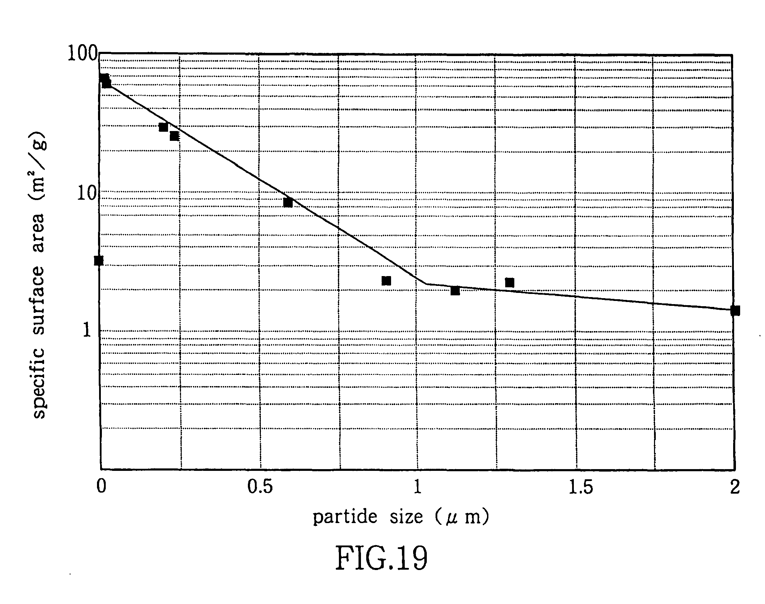

FIG.19 shows the relation between particle size and specific surface area of CuO. A specific surface area of 2 $m^2$/g or greater is equivalent to a particle size of less than about 1 $\mu$m.

**Measurement Example 4 (Comparative)**

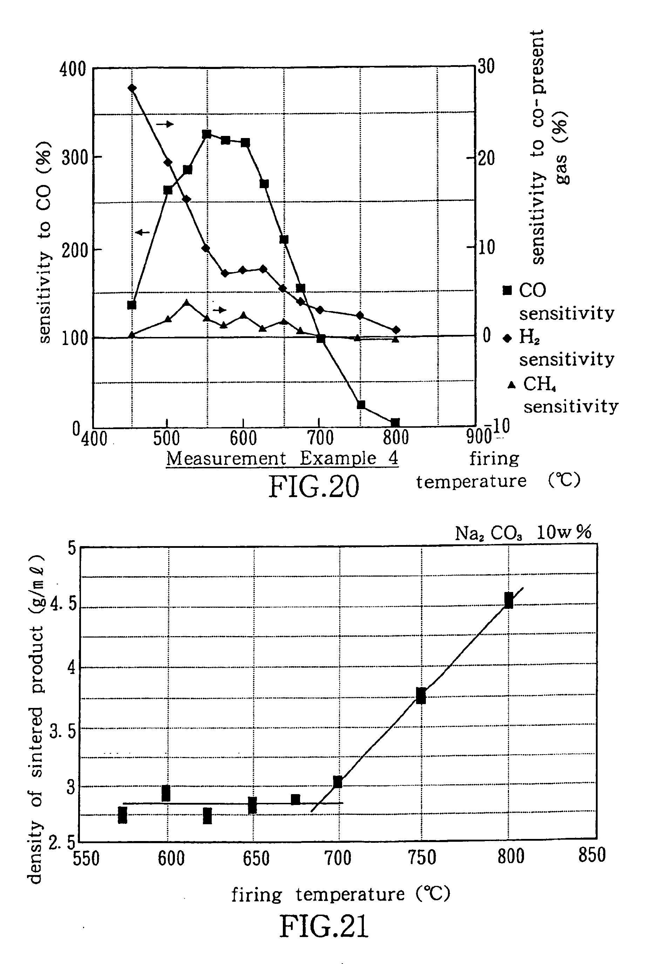

Gas sensors similar to that of Measurement Example 1 were fabricated using a variety of firing temperatures, and measurements made of variations in sensitivity ratio. An example of the results of these measurements is given in FIG.20. The sensors were fired after 10 wt% of $Na_2CO_3$ had been added to CuO having primary particles with a specific surface area of 52 $m^2$/g. CO, $H_2$ and $CH_4$ were used as the test gases, in each case at a concentration of 4000 ppm. The base gas was air. The vertical axis on the left-hand side of FIG.20 shows sensitivity to CO, while the vertical axis on the right-hand side shows sensitivity to the other co-present gas. When the firing temperature was 500°C to 700°C, sensitivity to CO was high, and more than ten times the sensitivity to $H_2$. However, the sensitivity to CO decreased when the firing temperature was outside this temperature range.

FIG.21 shows the relation between firing temperature when 10 wt% of $Na_2CO_3$ was added to CuO, and the density of the sintered product obtained under these conditions. It will be seen that density increases abruptly when firing temperature exceeds 700°C.

**Measurement Example 5**

A gas sensor with the construction shown in FIG.1 to Fig.3 was fabricated by cutting to shape, and attaching electrodes to, the sintered mass obtained by adding 8 wt% of $Na_2WO_4 \cdot 2H_2O$ to CuO and firing at a maximum temperature of 550°C. An alumina substrate was used as ceramic substrate 14.

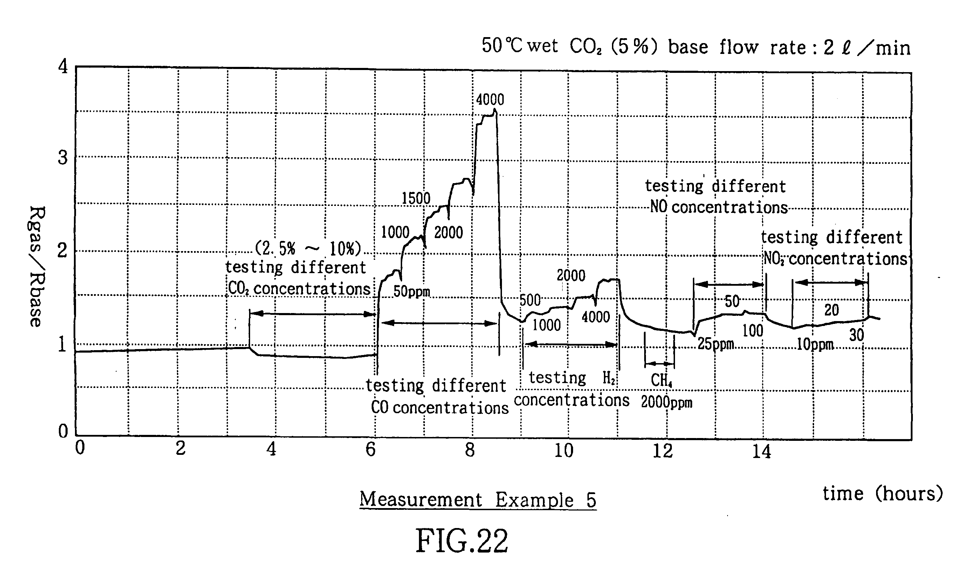

FIG.22 shows the results of measurements of sensitivity to various test gases. For these measurements, the apparatus illustrated in FIG.10 was used, and instead of being heated externally, gas sensor 40 being measured was heated by means of a heater provided on its rear surface. A voltage of 2.5 V was then applied between the electrodes and measurements made of the ratio of $R_{gas}$ to $R_{base}$ for a sequence of test gases, where $R_{gas}$ is the sensor resistance in the presence of a given test gas, and $R_{base}$ is the sensor resistance in the base condition. The base condition was an atmosphere comprising 5% $CO_2$ and the added water vapor equivalent of the partial pressure of saturated water vapor at 50°C and atmospheric pressure. $CO_2$, CO, $H_2$, $CH_4$, NO and $NO_2$ were used as the test gases. The measurements were made under two $CO_2$ conditions: a first in which only $CO_2$ was tested and the $CO_2$ concentration was varied within the range 2.5-10%, and a second in which another test gas was added to 5% $CO_2$. The test gases were used in the following concentrations: 500, 1000, 1500, 2000 and 4000 ppm for CO; 500, 1000, 2000 and 4000 ppm for $H_2$; 2000 ppm for $CH_4$; 25, 50 and 100 ppm for NO; and 10, 20 and 30 ppm for $NO_2$, and these gas concentrations were changed stepwise. It will be seen from the measurement results that there was hardly any sensitivity to $CO_2$, and that CO selectivity was very high.

## Measurement Example 6

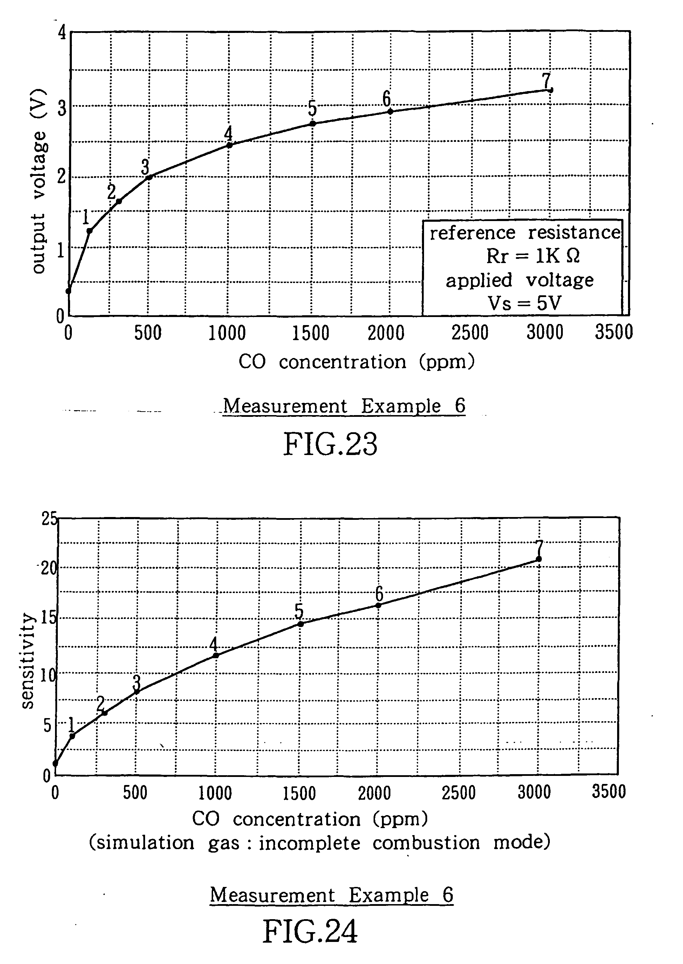

In order to measure sensor sensitivity during incomplete combustion in a water heater, gas sensors of the type used in Measurement Example 5 were used for measurements of sensitivity characteristics in a gas composition designed to simulate the exhaust gases resulting from incomplete combustion. The composition of the simulation gas is given in Table 1, and the results of the measurements are shown in FIG.23 and FIG.24. FIG.23 gives the results obtained when a voltage of 5 V was applied across a series-connected 1 kΩ reference resistance Rr and the gas sensor described above, and measurements made of the changes in sensor terminal voltage as a function of CO concentration. FIG.24 shows these measurement results as changes in sensitivity (i.e., the ratio of the resistance change) due to changes in CO concentration. The measurements of voltage change revealed that voltage changed considerably up to the onset of incomplete combustion (a state corresponding to a CO concentration of approximately 2000-4000 ppm), thereby indicating that measurement of voltage change is effective for detecting incomplete combustion. In practice, the output of the gas sensor can also be utilized after correction by a microprocessor or the like.

**TABLE 1**

| simulation gas | No. | NO ppm | $NO_2$ ppm | $SO_2$ ppm | $CH_4$ ppm | $CO_2$ % | $O_2$ % | CO ppm | $H_2$ ppm | water vapor mm Hg |
|---|---|---|---|---|---|---|---|---|---|---|
|  | 1 | 30 | 10 | 5 | 20 | 7 | 9.5 | 125 | 0 | 90 |
| normal | 2 | 40 | 15 | 5 | 10 | 7 | 8 | 300 | 65 | 90 |
|  | 3 | 50 | 15 | 5 | 5 | 8 | 7 | 500 | 200 | 90 |
|  | 4 | 60 | 15 | 5 | 5 | 8 | 6.5 | 1000 | 500 | 90 |
| incomplete | 5 | 60 | 15 | 5 | 5 | 8 | 6 | 1500 | 800 | 90 |
| combustion | 6 | 65 | 15 | 5 | 5 | 8.5 | 6 | 2000 | 1100 | 90 |
|  | 7 | 70 | 15 | 5 | 5 | 8.5 | 5 | 3000 | 2000 | 90 |

## Measurement Example 7

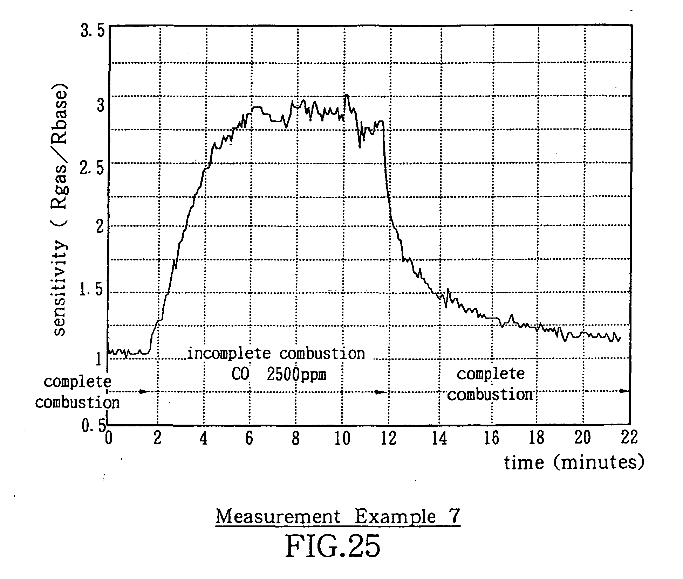

Sensor output characteristics during incomplete combustion in a forced draft type gas-fired water heater such as shown in FIG.6 were measured using gas sensors of the type employed in Measurement Examples 5 and 6. The results of these measurements are shown in FIG.25. The vertical axis shows the ratio of $R_{gas}$, sensor resistance during combustion, to $R_{base}$, sensor resistance when air is blown through the heater. Incomplete combustion is equivalent to a CO concentration of the order of 2500 ppm. FIG.25 shows that incomplete combustion can be detected by this gas sensor. Moreover, taking into consideration the measurement results of Measurement Examples 5 and 6 (FIG.23 and FIG.24), it will be seen that this gas sensor detects mainly CO gas.

## Measurement Example 8

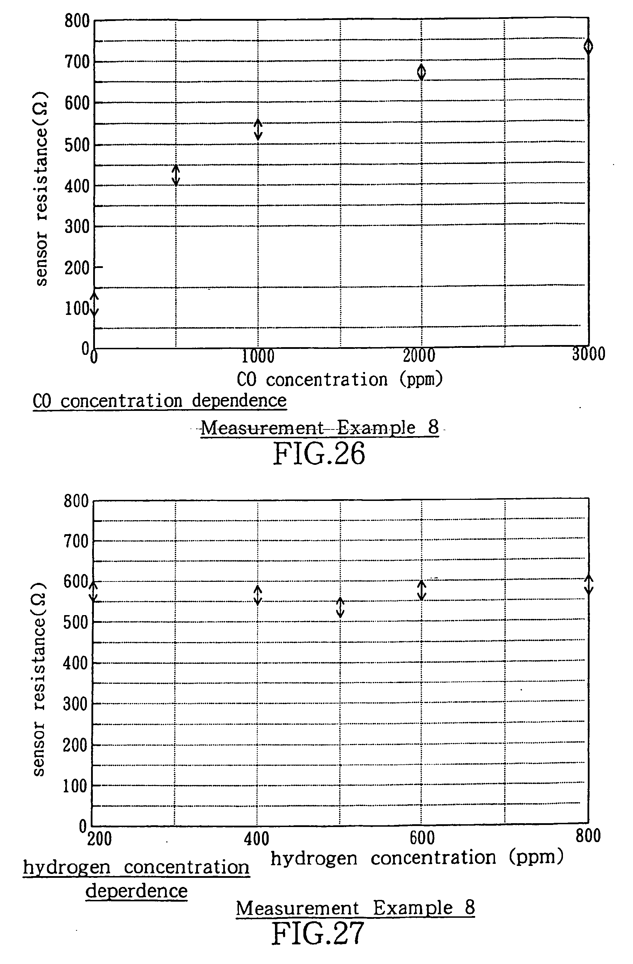

A 2 mm x 3 mm x 1 mm sintered mass was obtained by adding 8 wt% $Na_2WO_4 \cdot 2H_2O$ to CuO and firing at a maximum temperature of 600°C. Four gas sensors each having the structure shown in FIG.1 to FIG.3 were fabricated using this mass just as it was and attaching electrodes to the 2 mm x 3 mm faces. These four gas sensors were then used for measurements of sensor resistance as a function of the concentration of CO, $H_2$, $O_2$, water vapour and NO. The results are shown in FIG.26 to FIG.30. At any given concentration, the measured values obtained by the four gas sensors are nearly the same, and FIG.26 to FIG.30 show the range of these measured values.
FIG.26 shows sensor resistance as a function of CO concentration. For these measurements, the base gas was an atmosphere comprising $N_2$ to which 7.5% $CO_2$, 7.5% $O_2$, and the water vapour equivalent of the partial pressure of saturated water vapour at 50 °C and atmospheric pressure (12% water vapour) had been added. For a CO concentration at 500 ppm, the test gas used comprised 500 ppm of CO and 250 ppm of $H_2$. For a CO concentration of 1000 ppm, the test gas comprised 1000 ppm CO and 500 ppm $H_2$. For a CO concentration of 2000 ppm the test gas comprised 2000 ppm CO and 1000 ppm $H_2$, and for a CO concentration of 3000 ppm it comprised 3000 ppm CO and 1500 ppm $H_2$.

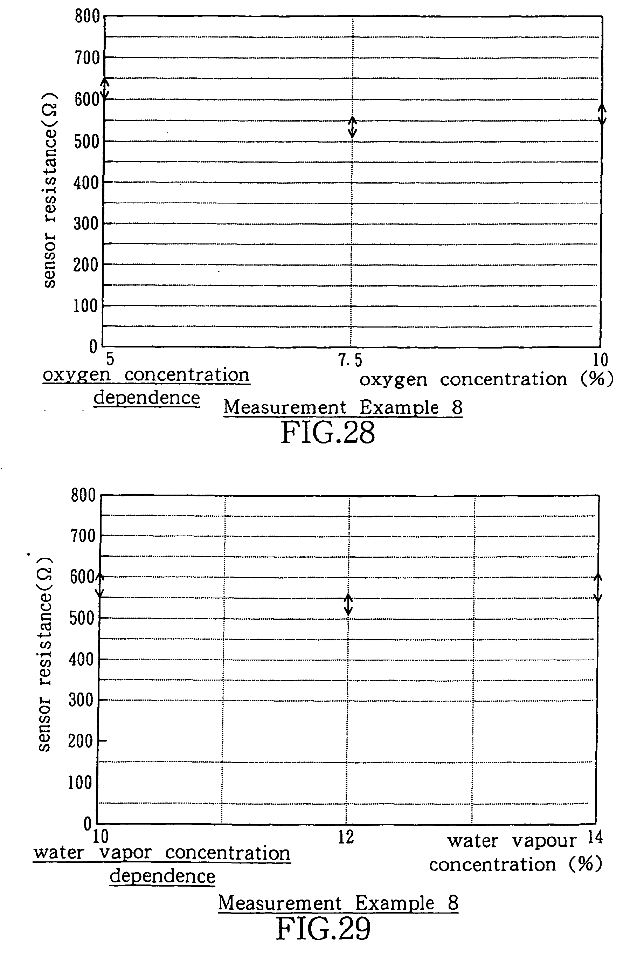

FIG.27 shows sensor resistance as a function of $H_2$ concentration. For these measurements, 1000 ppm of CO was added to a base gas comprising $N_2$ to which 7.5% $CO_2$, 7.5% $O_2$ and 12% water vapour had been added, and the

$H_2$ concentration was varied from 200 to 800 ppm.

FIG.28 shows sensor resistance as a function of $O_2$ concentration. For these measurements, 1000 ppm of CO and 500 ppm of $H_2$ were added to a base gas comprising $N_2$ to which 7.5% $CO_2$ and 12% water vapour had been added, and the $O_2$ concentration was varied from 5 to 10%.

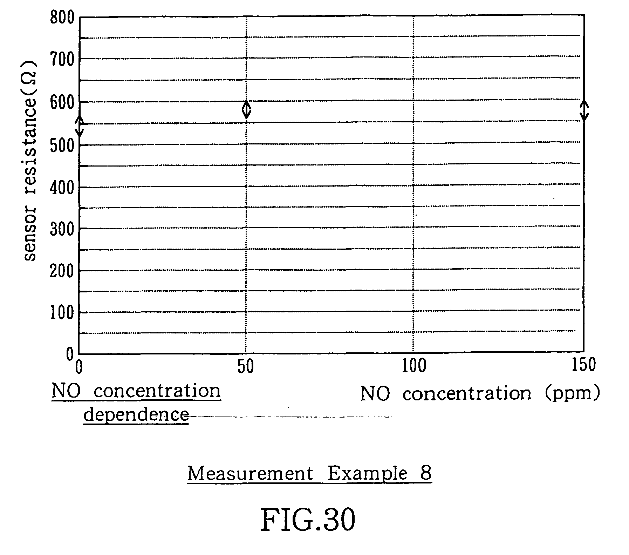

FIG.29 shows sensor resistance as a function of water vapour concentration. For these measurements, 1000 ppm of CO and 500 ppm of $H_2$ were added to a base gas comprising $N_2$ to which 7.5% $CO_2$ and 7.5% $O_2$ had been added, and water vapour was varied from 10 to 14%.

FIG.30 shows sensor resistance as a function of NO concentration. For these measurements, 1000 ppm of CO and 500 ppm of $H_2$ were added to a base gas comprising $N_2$ to which 7.5% $CO_2$, 7.5% $O_2$ and 12% water vapour had been added, and the NO concentration was varied from 0 to 150 ppm.

These measurement results show that the change in sensor resistance as a function of CO concentration is larger than its changes as functions of the concentration of $H_2$, $O_2$, water vapour and NO, thereby indicating suitability for detection of CO gas.

**Measurement Example 9**

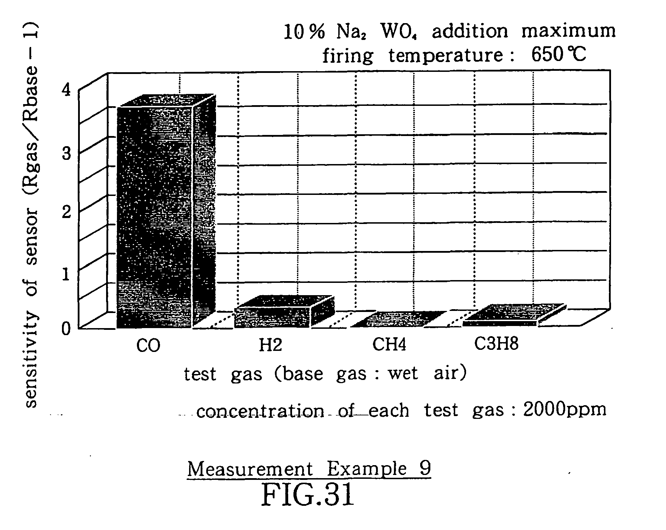

10 wt% of $Na_2WO_4 \cdot 2H_2O$ was added to CuO and the resulting mixture fired at a maximum temperature of 650°C. A gas sensor with the same structure as in Measurement Example 5 was fabricated by cutting the sintered mass obtained and attaching electrodes. The sensitivity of this gas sensor to CO, $H_2$, $CH_4$ and $C_3H_8$ was then measured. The results are shown in FIG.31. The base gas used in these measurements was dry air to which water vapour equivalent to saturated water vapour at 25°C and atmospheric pressure had been added, and the measurements were made with the concentration of each gas set at 2000 ppm. The measurement results given in FIG.31 show that this gas sensor has a high degree of selectivity for CO gas.

**Measurement Example 10**

A thick film gas sensor with the structure shown in FIG.4 and FIG.5 was fabricated by forming a paste comprising 5 wt% of $Na_2WO_4$ (anhydride) added to CuO, screen printing this onto interdigitated electrodes, and firing at a maximum temperature of 650°C. An alumina substrate was used as ceramic substrate 24, and a heater for heating the sensor was provided on the rear surface.

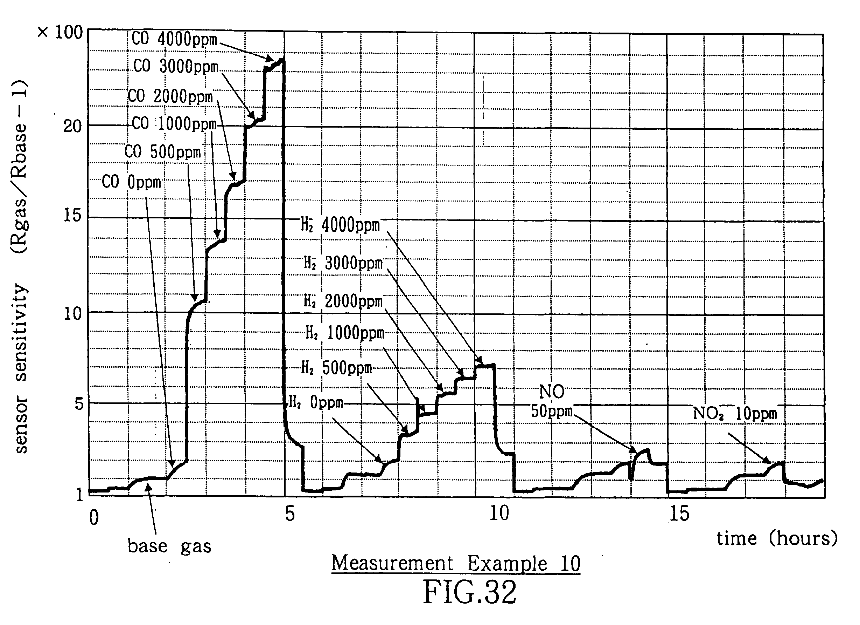

FIG.32 shows the results of measurements of sensitivity to various test gases. These measurements were performed in the same manner as in Measurement Example 5, using 7.5% $CO_2$, 7.5% $O_2$ and 12% water vapour added to $N_2$ as the base gas, and 500, 1000, 2000, 3000 and 4000 ppm of CO; 500, 1000, 2000, 3000 and 4000 ppm of $H_2$; 50 ppm of NO, and 10 ppm of $NO_2$ as the test gases. These measurement results show that the CO selectivity is very high.

**Measurement Example 11**

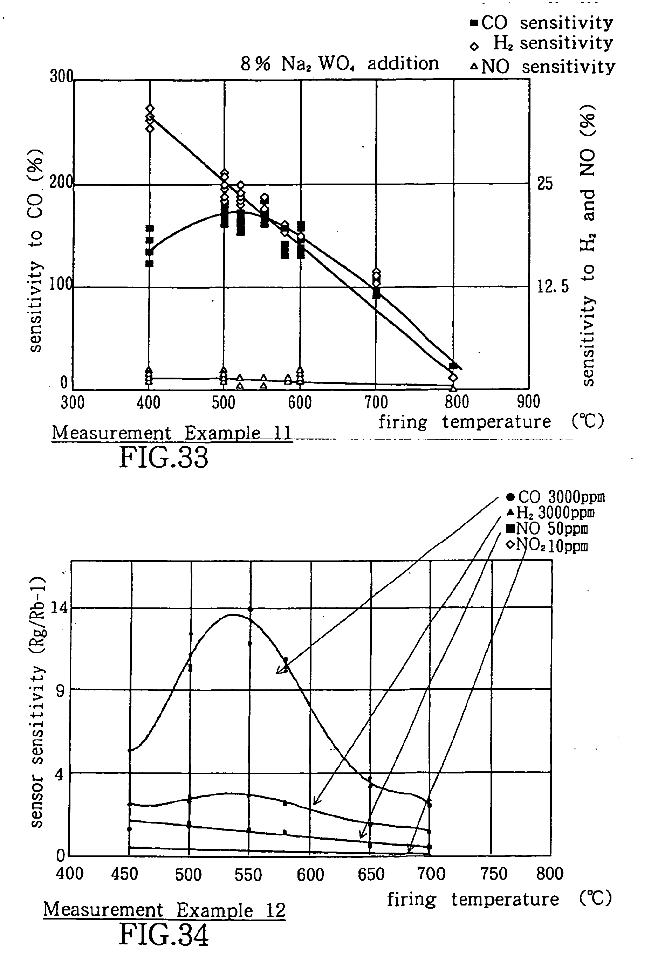

Gas sensors with the same structure as in Measurement Example 1 (see FIG.12 to FIG.14) were fabricated by adding 8 wt% of $Na_2WO_4 2H_2O$ to CuO and firing at a variety of firing temperatures. Measurements were then made of gas sensitivity as a function of maximum firing temperature. The results are shown in FIG.33. Air with a $CO_2$ concentration of 5% was used as the base gas, and 4000 ppm of CO, 4000 ppm of $H_2$ and 50 ppm of NO were used as the test gases. The vertical axis on the left-hand side of FIG.33 shows CO sensitivity, while the vertical axis on the right-hand side shows $H_2$ sensitivity and NO sensitivity. A satisfactorily high CO sensitivity is obtained if the maximum firing temperature is 400°C or over, but there is a conspicuous decrease in CO sensitivity if the maximum firing temperature exceeds 500°C (at which temperature the strength of the sintered mass is high). Provided that the maximum firing temperature is no more than 800°C, the CO sensitivity is higher than the $H_2$ sensitivity.

**Measurement Example 12**

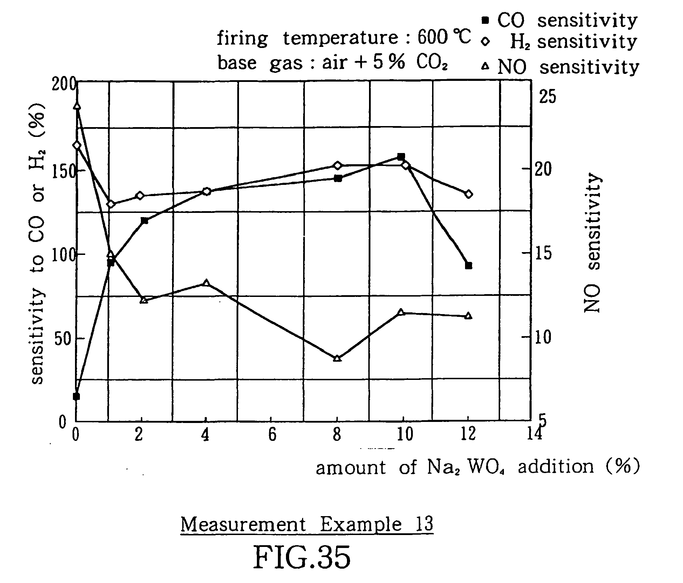

Thick film gas sensors of the same construction as in Measurement Example 10 were fabricated using a paste comprising 5 wt% $Na_2WO_4$ (anhydride) added to CuO, and employing a variety of firing temperatures. Measurements were then made of gas sensitivity as a function of maximum firing temperature. The results are shown in FIG.34. $N_2$ to which 7.5% $CO_2$, 7.5% $O_2$ and 12% water vapour had been added was used as the base gas, and 3000 ppm of CO, 3000 ppm of $H_2$, 50 ppm of NO and 10 ppm of $NO_2$ were used as the test gases. It will be seen from these measurement results that satisfactorily high CO sensitivity is obtained if the firing temperature is 450°C or over, but that there is a pronounced decrease in CO sensitivity if the firing temperature exceeds 550°C. Provided that the firing temperature is no more than 700°C, the CO sensitivity is higher than the $H_2$ sensitivity.

**Measurement Example 13**

Gas sensors were fabricated with different amounts of $Na_2WO_4\cdot2H_2O$ addition, and their sensitivity measured. The maximum firing temperature was set at 600°C. The structure of the gas sensors was the same as in Measurement Example 12 (see FIG.12 to FIG.14). The results of these measurements are shown in FIG.35. Air with a $CO_2$ concentration of 5% was used as the base gas, and 4000 ppm of CO, 4000 ppm of $H_2$, and 50 ppm of NO were used as the test gases. The vertical axis on the left-hand side of FIG.35 shows the CO sensitivity and $H_2$ sensitivity, while the vertical axis on the right-hand side shows NO sensitivity. It will be seen from FIG.35 that CO sensitivity increases remarkably when the amount of addition of the $Na_2WO_4\cdot2H_2O$ is 2 wt% or more.

**Measurement Example 14**

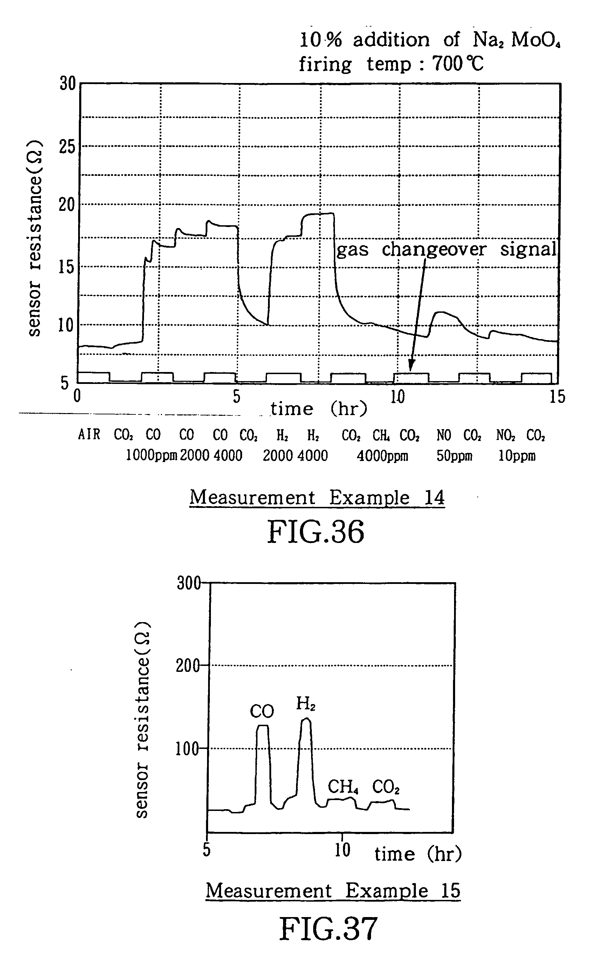

A gas sensor was fabricated by adding 10 wt% of $Na_2MoO_4\cdot2H_2O$ to CuO, firing at a maximum temperature of 700°C, and attaching electrodes to the sintered mass thereby obtained. Measurements were then made of the resistance of this sensor. The structure of the sensor was the same as in Measurement Example 12 (see FIG.12 to FIG.14). The measurement results are shown in FIG.36. Air with a $CO_2$ concentration of 5% was used as the base gas, and 1000, 2000 and 4000 ppm of CO, 2000 and 4000 ppm of $H_2$, 4000 ppm of $CH_4$, 50 ppm of NO, and 10 ppm of $NO_2$ were used as the test gases. An initial measurement was also made for air only, and it was found that there was a small change in sensor resistance according to whether $CO_2$ was present or not. Although the $H_2$ sensitivity of this gas sensor is close to the CO sensitivity, it is fully capable of detecting actual incomplete combustion.

**Measurement Example 15**

A gas sensor with the same structure as in Measurement Example 5 (see FIG.1 to FIG.3) was fabricated from a sintered mass which had been fired under the same conditions as Measurement Example 14, and measurements were made of its resistance. The results of these measurements are given in FIG.37. Air was used as the base gas, and 4000 ppm of CO, 4000 ppm of $H_2$, 4000 ppm of $CH_4$ and 5% $CO_2$ were used as the test gases.

**Measurement Example 16**

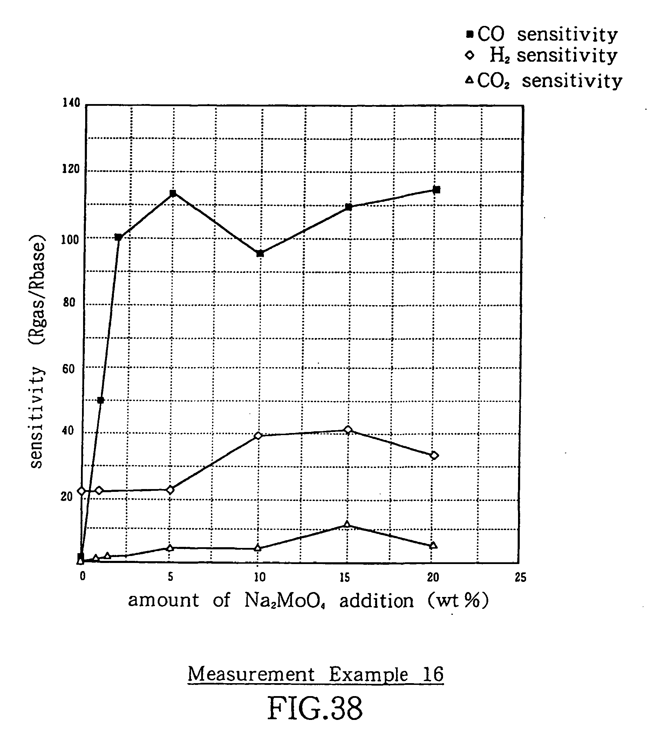

Gas sensors were fabricated with different amounts of $Na_2MoO_4\cdot2H_2O$ addition, and measurements made of the resulting changes in sensitivity. The maximum firing temperature was set at 600°C and the structure of the sensors was the same as in Measurement Example 12 (see FIG.12 to FIG.14). The results of these measurements are shown in FIG.38. Air was used as the base gas, and 4000 ppm of CO, 4000 ppm of $H_2$, and 5% $CO_2$ were used as the test gases. These measurement results show that CO sensitivity increases remarkably when the amount of addition is greater than 1 wt%.

**Claims**

1.   A gas sensor having:

 a p-type member (11;21) formed from a p-type semiconductor the main constituent of which is CuO and to which a sodium compound has been added; and
 two electrodes (12, 13; 22, 23) connected to this p-type member, said electrodes serving to extract changes in electrical characteristics resulting from the presence of a gas to be detected;
 **characterised in that** the sodium compound is a sodium salt of tungstic acid at from 0.5 to 23 wt% as tungsten relative to CuO, or of molybdic acid at from 0.4 to 16 wt% as molybdenum relative to CuO.

2.   A method for manufacturing a gas sensor by forming a member with electrical characteristics which change in accordance with the presence of a gas to be detected, said member being formed by adding a sodium compound to CuO and then moulding and firing;
 **characterised in that** the sodium compound is a sodium salt of tungstic acid at from 0.5 to 23 wt% as tungsten relative to CuO, or of molybdic acid at from 0.4 to 16 wt% as molybdenum relative to CuO.

3.   A method for manufacturing a gas sensor according to claim 2, wherein $Na_2WO_4\cdot2H_2O$ is added at 1 to 40 wt% relative to CuO.

4. A method for manufacturing a gas sensor according to claim 2, wherein $Na_2MoO_4 \cdot 2H_2O$ is added at 1 to 40 wt% relative to CuO.

5. A method for manufacturing a gas sensor according to any of claims 2 to 4, wherein the maximum temperature during firing is at least 400 °C.

6. A method for manufacturing a gas sensor according to claim 5, wherein the maximum temperature during firing is no more than 860 °C.

7. A method for manufacturing a gas sensor according to claim 6, wherein the maximum temperature during firing is at least 500 °C and no more than 850 °C.


**Patentansprüche**

1. Gassensor, aufweisend:

   ein p-Typ-Element (11; 21), das aus einem p-Typ-Halbleiter gebildet ist, dessen Hauptbestandteil CuO ist und dem eine Natriumverbindung zugefügt wurde; und

   zwei Elektroden (12, 13; 22, 23), die mit diesem p-Typ-Element verbunden sind, wobei die Elektroden dazu dienen, Änderungen bei den elektrischen Eigenschaften, die sich aus der Gegenwart eines zu detektierendem Gas ergeben, zu extrahieren;

   **dadurch gekennzeichnet, dass** die Natriumverbindung ein Natriumsalz von Wolframsäure mit von 0,5 bis 23 Gew.-% Wolfram relativ zu CuO oder von Molybdänsäure mit von 0,4 bis 16 Gew.-% Molybdän relativ zu CuO ist.

2. Verfahren zum Herstellen eines Gassensors durch Bilden eines Elements mit elektrischen Eigenschaften, die sich in Übereinstimmung mit der Gegenwart eines zu detektierenden Gases ändern, wobei das Element gebildet wird durch Zufügen einer Natriumverbindung zu CuO und anschließende Formgebung und Brennen;

   **dadurch gekennzeichnet, dass** die Natriumverbindung ein Natriumsalz von Wolframsäure mit von 0,5 bis 23 Gew.-% Wolfram relativ zu CuO oder von Molybdänsäure mit von 0,4 bis 16 Gew.-% Molybdän relativ zu CuO ist.

3. Verfahren zum Herstellen eines Gassensors nach Anspruch 2, wobei $Na_2WO_4 \cdot 2H_2O$ mit 1 bis 40 Gew.-% relativ zu CuO zugefügt wird.

4. Verfahren zum Herstellen eines Gassensors nach Anspruch 2, wobei $Na_2MoO_4 \cdot 2H_2O$ mit 1 bis 40 Gew.-% relativ zu CuO zugefügt wird.

5. Verfahren zum Herstellen eines Gassensors nach einem der Ansprüche 2 bis 4, wobei die maximale Temperatur während des Brennens mindestens 400°C ist.

6. Verfahren zum Herstellen eines Gassensors nach Anspruch 5, wobei die maximale Temperatur während des Brennens nicht mehr als 860°C ist.

7. Verfahren zum Herstellen eines Gassensors nach Anspruch 6, wobei die maximale Temperatur während des Brennens mindestens 500°C und nicht mehr als 850°C ist.


**Revendications**

1. Détecteur de gaz possédant:

   un élément de type p (11; 21) formé d'un semiconducteur de type p, dont le constituant principal est CuO et auquel a été ajouté un composé de sodium; et

   deux électrodes (12, 13; 22, 23) connectées à cet élément de type p, lesdites électrodes servant à extraire des modifications de caractéristiques électriques résultant de la présence d'un gaz à détecter;

   **caractérisé en ce que** le composé de sodium est un sel de sodium de l'acide tungstique comprenant de 0,5 à 23 % en poids de tungstène par rapport au CuO, ou de l'acide molybdique comprenant de 0,4 à 16 % en poids de molybdène par rapport au CuO.

**2.** Procédé pour fabriquer un détecteur de gaz par formation d'un élément présentant des caractéristiques électriques, qui varient en fonction de la présence d'un gaz à détecter, ledit élément étant formé par addition d'un composé de sodium au CuO, puis moulage et cuisson;
**caractérisé en ce que** le composé de sodium est un sel de sodium de l'acide tungstique comprenant de 0,5 à 23 % en poids de tungstène par rapport au CuO, ou de l'acide molybdique comprenant de 0,4 à 16 % en poids de molybdène par rapport au CuO.

**3.** Procédé pour fabriquer un détecteur de gaz selon la revendication 2, dans lequel du $Na_2WO_4.2H_2O$ est ajouté en tant que 1 à 40 % en poids par rapport au CuO.

**4.** Procédé pour fabriquer un détecteur de gaz selon la revendication 2, dans lequel du $Na_2MoO_4.2H_2O$ est ajouté en tant que 1 à 40 % en poids par rapport au CuO.

**5.** Procédé pour fabriquer un détecteur de gaz selon l'une quelconque des revendications 2 à 4, selon lequel la température maximale pendant la cuisson est égale à au moins 400°C.

**6.** Procédé pour fabriquer un détecteur de gaz selon la revendication 5, selon lequel la température maximale pendant la cuisson n'est pas supérieure à 860°C.

**7.** Procédé pour fabriquer un détecteur de gaz selon la revendication 6, selon lequel la température maximale pendant la cuisson est égale à au moins 500°C et n'est pas supérieure à 850°C.

Plan View of First Embodiment

## FIG.1

Side View of First Embodiment

## FIG.2

Rear Plan View of First Embodiment

## FIG.3

Plan View of Second Embodiment

FIG.4

Sectional View of Second Embodiment

FIG.5

Flue

CO Sensor Filted
somewhere here

Burner

Blower

Combustion
Products

Outside

Combustion Air

Mode of use

FIG.6

Blower for Exhaust

Flue

Blowback Preventer

CO Sensor Filted somewhere here

Combustion Air→

Flue Terminal

Combustion Products

Air Supply Vent

Mode of use

FIG.7

Intake/Exhaust Flue

Combustion Products

Outside

Combustion Air

CO Sensor Fitted in Pipe Projecting Inside Flue

Room

Mode of use

FIG.8

Intake/Exhaust
Flue

CO Sensor Fitted in
Pipe Projecting Inside
Flue

Combustion
Products

Outside

Room

Combustion
Air

Mode of use
FIG.9

Air
CO – h
CO – m
CO – l
H2
C3H8
NOx

41

Selenoid
Valves

42

Flowmeter

43

40

Exhaust

Temperature
Controller

44

45

49

Relays

Multimeter

48

Controller

46

47

Apparatus for Measuring Sensitivity
FIG.10

Example of Measurement Results (prior art)

FIG.11

Gas Sensor used for Measurements

FIG.12

31

32                    33

34                    35

Side View

FIG.13

31

Electrode                    33

Lead Wire

View Facing One of the Electrodes

FIG.14

Na₂ CO₃ 10w %

Measurement Example 1

FIG.15

- CO sensitivity
- ◇ H₂ sensitivity
- ▲ CH₄ sensitivity

amount of Na₂CO₃ addition (wt %)

Measurement Example 2

FIG.16

(comparison gases)

Measurement Example 3

FIG.17

FIG.18

■ CO sensitivity ◇ NO₂ sensitivity
◆ H₂ sensitivity △ SO₂ sensitivity
▲ CH₄ sensitivity ● CO₂ sensitivity
NO sensitivity

EP 0 834 738 B1

FIG.19

EP 0 834 738 B1

Measurement Example 4

FIG.20

FIG.21

FIG.22

Measurement Example 6

FIG.23

(simulation gas : incomplete combustion mode)

Measurement Example 6

FIG.24

Measurement Example 7

FIG.25

CO concentration dependence

Measurement Example 8

FIG.26

hydrogen concentration
deperdence

Measurement Example 8

FIG.27

oxygen concentration dependence   Measurement Example 8

FIG.28

water vapor concentration dependence   Measurement Example 8

FIG.29

NO concentration
   dependence

Measurement Example 8

FIG.30

Measurement Example 9

# FIG.31

29

Measurement Example 10

FIG.32

EP 0 834 738 B1

8 % Na₂ WO₄ addition

- ■ CO sensitivity
- ◇ H₂ sensitivity
- △ NO sensitivity

Measurement Example 11

FIG.33

- ● CO 3000ppm
- ▲ H₂ 3000ppm
- ■ NO 50ppm
- ◇ NO₂ 10ppm

Measurement Example 12

FIG.34

Measurement Example 13

# FIG.35

10 % addition of Na₂ MoO₄
firing temp : 700℃

Measurement Example 14

FIG.36

Measurement Example 15

FIG.37

Measurement Example 16

FIG.38